Europäisches Patentamt

**European Patent Office**

**Office européen des brevets**

(11) Publication number: **0 200 095**
**B1**

(12) **EUROPEAN PATENT SPECIFICATION**

(45) Date of publication of patent specification: **29.08.90**

(21) Application number: **86105255.3**

(22) Date of filing: **16.04.86**

(51) Int. Cl.⁵: **C 12 N 11/08, C 12 P 19/22**

(54) Immobilized maltooligosaccharide-forming amylase and process for the production of maltooligosaccharide using said enzyme.

(30) Priority: **18.04.85 JP 81340/85**
**11.06.85 JP 125093/85**

(43) Date of publication of application:
**05.11.86 Bulletin 86/45**

(45) Publication of the grant of the patent:
**29.08.90 Bulletin 90/35**

(84) Designated Contracting States:
**DE FR GB NL**

(56) References cited:
**EP-A-0 110 574**
**EP-A-0 112 147**

The file contains technical information submitted after the application was filed and not included in this specification

(73) Proprietor: **The Japanese Research and Development Association for Bioreactor System**
Honda Bldg. 2-13, 3-chome, Toranomon
Minato-ku Tokyo (JP)

(72) Inventor: **Kimura, Takashi**
18-20, 3-chome, Konandai Konan-ku
Yokohama-shi Kanagawa-ken (JP)
Inventor: **Noguchi, Masaaki**
20-3, 2-chome, Nakahara
Mitaka-shi Tokyo (JP)
Inventor: **Ogata, Masafumi**
No. 325, Shinsaku Takatsu-ku
Kawasaki-shi Kanagawa-ken (JP)
Inventor: **Kobayashi, Haruto**
12-11, 2-chome, Hashido Seya-ku
Yokohama-shi Kanagawa-ken (JP)
Inventor: **Miwa, Taizo**
No. 2954, Imaizumi
Fuji-shi Shizuoka-ken (JP)
Inventor: **Nakakuki, Teruo**
Kamo Green Hill No. 7 No. 57, Kamo
Mishima-shi Shizuoka-ken (JP)
Inventor: **Yoshida, Masahiro**
No. 2954, Imaizumi
Fuji-shi Shizuoka-ken (JP)

Courier Press, Leamington Spa, England.

**EP  0 200 095  B1**

⁷⁴ Representative: **Türk, Dietmar, Dr. rer. nat. et al
Türk, Gille + Hrabal Patentanwälte
Brucknerstrasse 20
D-4000 Düsseldorf 13 (DE)**

## Description

The present invention relates to an immobilized maltooligosaccharide-forming amylase, selected from the group consisting of maltotriose-forming amylase, maltotetraose-forming amylase, maltopentaose-forming amylase and maltohexaose-forming amylase, which is immobilized on a porous carrier wherein the carrier is porous synthetic resin selected from the group consisting of phenol-based absorption resin, styrene/divinylbenzene-based adsorption resin, triazine-based adsorption resin, and acrylate-based adsorption resin and a process for the production of maltooligosaccharide using said enzyme by feeding starch hydrolizate (liquefied starch) to a reactor charged with the above immobilized enzyme, under specific conditions.

Decomposition of starch or amylose with acid or amylase results in the formation of various oligosaccharide mixtures. These mixtures are produced industrially and consumed in large quantities as starch saccharides such as starch syrup and powder. It has been difficult, however, to produce high purity maltooligosaccharides, except for maltose, on commercial scale and with high efficiency.

In recent years, various maltooligosaccharide-forming amylases specifically producing maltooligosaccharides such as maltotriose, maltotetraose, maltopentaose and maltohexaose on acting on starch or amylose have been discovered. Thus, efficient production of maltooligosaccharides using such specific amylases on a commercial scale has been expected.

As maltooligosaccharide-forming amylases, as well as beta (β)-amylase, the followings are known.

Maltotriose-forming amylase (from *Streptomyces griseus*, as described by K. Wako et al. in J. Jpm. Soc. Starch Sci., *26*, 175 (1979) and from *Bacillus* sp., as described by Y. Takasaki in Abstract Paper of the Annual Meeting of the Agricultural Chemical Society of Japan, page 169 (1983)).

Maltotetraose-forming amylase (from *Pseudomonas stutzeri*, as described by J. F. Robyt and R. J. Ackerman in Arch. Biochem. Biophys., *145*, 105 (1971)).

Maltopentaose-forming amylase (from *Bacillus licheniformis*, as described by N. Saito in Arch. Biochem. Biophys., *155*, 290 (1973), by S. Kobayashi et al. in Abstract Paper of the Annual Meeting of the Japanese Society of Starch Science, page 301 (1983), and by Yoshigi et al. in Abstract Paper of the Annual Meeting of the Agricultural Chemical Society of Japan, page 584 (1984)).

Maltohexaose-forming amylase (from *Aerobacter aerogenes*, as described by K. Kainuma et al. in FEBS Lett., *26*, 281 (1972), by J. F. Kennedy and C. A. White in Stärke, *31*, 93 (1979), by Taniguchi et al. in J. Jpm. Soc. Starch Sci., *29*, 107 (1982), and by Y. Takasaki, in Agric. Biol. Chem., *47*, 2193 (1983)).

These maltooligoschaccharide-forming amylases, however, are quite expensive as compared with general amylases, and only small amounts of reagent grade of maltooligosaccharides are produced using the above specific amylases. These reagent grade of maltooligosaccharides are produced by batch process, and thus various problems have been encountered. For example, a reaction time as long as 24 to 72 hours is needed. The maltooligosaccharide-forming amylase were discarded every batchwise reaction. In addition to the separation of the objective maltooligosaccharide from other saccharides, it is necessary to separate the product from the used enzyme, and thus the purification/separation process becomes complicated and needs much man-hour.

It has been revealed, as described by S. Suzuki and M. Nakamura in "Experimental Method for Starch Chemistry", Asakura Shoten, page 197—202, that purification techniques such as the gel filtration method, carbon column chromatography, etc. are unsuitable for mass-production because the processing load is low and furthermore productivity is low. Therefore, in order to commercially produce various oligosaccharides using the above maltooligosaccharide-forming amylases, it is essential to efficiently utilize the amlyases. For this purpose, it is considered to immobilize the amlyases.

For immobilization of the specific amylase to produce specific maltooligosaccharide having a higher degree maltose than maltotriose, there has been known only a method in which a maltohexaose-forming amylase is immobilized on polyacrylamide gel by the irradiation method (T. Nakakuki et al, Biotechnol. and Bioeng., *25*, page 1095—1107 (1983)). This method, however, has disadvantages in that although an enzyme immobolization ratio is high, the method is unsuitable for mass-production, the method is expensive, and the activity per weight of the immobilized enzyme is low.

Therefore the present inventors have made extensive investigations on immobilization in which one of the above maltooligosaccharide-forming amylase is immobilized with high yield, the activity per weight of which is sufficiently high for commercial use, and which can maintain its activity for long periods of time, and furthermore, on a process for the production of the said maltooligosaccharide using this immobilized enzyme.

As a result, it has been found that the desired excellent immobilized enzyme can be prepared by using a specific porous carrier. Furthermore it has been found that maltooligosaccharide can be efficiently produced by feeding starch hydrolysates to a reactor charged with immobilized enzyme, under specific conditions as determined based on the activity of the immobilized enzyme.

The present invention, in one embodiment, relates to an above mentioned immobilized maltooligosaccharide-forming amylase having the following characteristics:

(1) the affinity of the enzyme to the carrier is at least 80%; and

(2) the specific surface area of pores having a pore diameter of at least $10^{-8}$ m is from 20 to 300 square meters per gram (m²/g).

EP 0 200 095 B1

In the other embodiment, the present invention relates to a process for producing a maltooligosaccharide which comprises feeding starch hydrolysates to a reactor charged with the above immobilized maltooligosaccharide-forming amylase, under such conditions that the weight based space velocity per unit activity of the immobilized enzyme is $1 \times 10^{-4}$ to $3 \times 10^{-2}$ hr$^{-1}$ (IU/g)$^{-1}$.

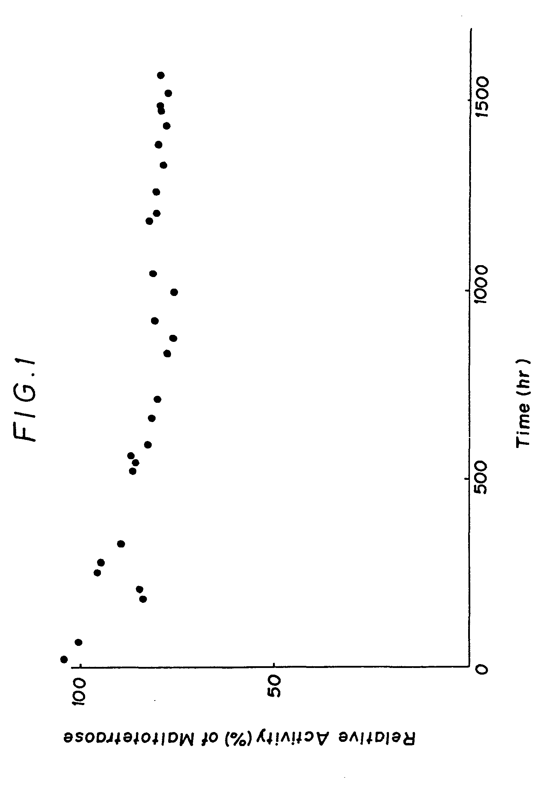

The Figure shows a change on relative activity of an immobilized enzyme with time.

Various methods are known for immobilization of enzyme. Typical methods are carrier-bonding methods, which include a physical adsorption method, an ionic bonding method and a covalent bonding method. Of the above methods, the physical adsorption and ionic bonding methods have advantages in that the preparation is simple, they less break the active center and three dimensional configuration of enzyme protein, and furthermore in that regeneration of the carrier is easy. Thus these methods are desirable for commercial use as long as a good carrier can be discovered.

In the present invention, as a carrier for maltooligosaccharide-forming amylases, a porous carrier having the following characteristics are used:

(1) the affinity of the enzyme to the carrier is at least 80%; and

(2) the specific surface area of pores having a pore diameter of at least $10^{-8}$ m is 20 to 300 m$^2$/g.

The affinity of the enzyme to the carrier is measured by the following method.

Exactly 0.5 gram (g) of a carrier and 2.0 milliliters (ml) solution of a 50 millimole (mM) phosphate buffer (pH: 7.0)) 25 IU of an enzyme are placed in a 50-milliliter Erlenmeyer flask, which is then shaked for 1 hour at room temperature under conditions of 120 strokes per minute and 4 centimeter (cm) width to thereby immobilize the enzyme on the carrier. Then the resulting mixture is filtrated, and the activity of the enzyme in the filtrate is measured. The affinity of the enzyme to the carrier is determined according to the following equation:

$$\frac{\text{Activity of added enzyme} - \text{Activity of enzyme in filtrate}}{\text{Activity of added enzyme}} \times 100$$

Although the purity of an enzyme for use in the measurement of affinity is not critical, it is preferred to use an enzyme having a specific activity of at least 30 IU/mg protein. Although the above affinity is measured at an enzyme activity/carrier weight ratio which is lower than that commercially used, it is important as a measure of determining the affinity. That is, the present inventors have confirmed that when an enzyme immobilized at a high enzyme activity/carrier weight ratio exhibits high activity for a substrate, it has a high affinity value as determined by the above equation.

It is necessary for the affinity as determined above to be not less than 80%. In the case of a carrier for maltotetraose-forming amylase, the affinity is preferably not less than 95%, and in the case of a carrier for maltopentaose-forming amylase, the affinity is preferably not less than 80%. These values are determined by the origin and specific activity of the enzyme used.

The term "exhibited activity" is defined as follows:

Activity of immobilized enzyme greatly varies with the conditions under which the enzyme is used. Therefore the activity is assayed under the following conditions. That is, 10 mg (wet) of an immobilized enzyme is added to 0.5 ml of a 10 mM phosphate buffer (pH: 7.0) in a 50 ml Erlenmeyer flask and mixed thoroughly. Then, 5.0 ml of 10 w/v % soluble starch (produced by Merck Co.) is added, and the flask is shaken at 40°C for 10 minutes by means of a reciprocal shaker under the conditions of 120 strokes/min and 4 cm width to conduct an enzyme reaction. The reducing sugar thus formed is measured for by the Somogyi-Nelson method to determine the immobilized enzyme activity. One International Unit (IU) as defined according to the International Commission on Enzyme in the amount of enzyme that hydrolyzes one micromole of glucosidic bonds per minute under there conditions.

The carrier which is used in the present invention is further required to meet the following condition. That is, the specific surface area of pores having a pore diameter of not less than $10^{-8}$ m is 20 to 300 square meters per gram (m$^2$/g) and preferably 40 to 200 m$^2$/g. If the specific surface area is in excess of 300 m$^2$/g, the strength of the carrier is undesirably poor. In the case of a carrier for maltotetraose-forming amylase, the specific surface area of pores having a pore diameter of not less than $4 \times 10^{-8}$ m is 3 to 100 m$^2$/g and preferably 5 to 50 m$^2$/g. The specific surface area is measured by the mercury-press-injection method.

Carriers meeting only one of the above requirements are unsatisfactory; that is, carriers meeting all the requirements exhibit high activity to a substrate.

The porous carrier that is used in the present invention is not critical in material, method of preparation and so forth as long as it satisfies the above requirements. Usually the above mentioned porous synthetic resins such as phenol-based adsorption resin, styrene/divinylbenzene-based adsorption resin, triazine-based adsorption resin, and acrylate-based adsorption resin are used. More specifically the followings can be used.

Duolite S-761, S-762, ES-771, ES-8610 (trade names, porduced by Diamond Shamlock Co., Ltd.), Diaion HP-10, HP-20, HP-50, SP-206 (trade names, porduced by Mitsubishi Chemical Industries Co., Ltd.), and Amberlite XAD-7, XAD-8 (trade names, produced by Rhome and Haas Co., Ltd.).

There are no special limitations to the particle diameter of the porous carriers as long as its exhibited activity is high. The particle diameter is usually 0.05 to 1.5 millimeters (mm) and preferably 0.2 to 1.0 mm.

4

From viewpoints of immobilization and exhibited activity of the enzyme, it is desirable that the particle diameter is small. However, if the particle diameter is too small, when the immobilized enzyme is placed in a reactor and starch hydrolisates is introduced therein, the pressure drop is large. This is undesirable from a viewpoint of operation. On the other hand, if the particle diameter is too large, the efficiency of immobilization of the enzyme is low and exhibited activity is poor.

Immobilization of these enzymes can be carried out by various methods. The most popular method is, as described above, to dissolve the enzyme in a suitable buffer and then to mix and stir the resulting solution with a carrier. Thereafter solid-liquid separation is carried out by techniques such as filtration and centrifugation, and the immobilized enzyme thus obtained is washed with a suitable buffer. Suitable washing solutions which can be used for the above puprose are a phosphate buffer (pH 6—7), a tris-hydrochloric acid buffer (pH 7—9) and distilled water.

The amount of the enzyme immobilized is usually from 1 to 40 mg (calculated as protein) per gram of the carrier and preferably from 2 to 15 mg (calculated as protein) per gram of the carrier.

If the amount of the enzyme immobilized is less than 1 mg (calculated as protein) per gram of the carrier, the immobilization ratio is high; the enzyme can be immobilized efficiently, but the activity is low and the yield of the desired maltooligosaccharide component is low. On the other hand, if the amount of the enzyme immobilized is in excess of 40 mg (calculated as protein) per gram of the carrier, the immobilization ratio is decreased and, therefore, not only the efficiency of use of the enzyme is poor but also the exhibited activity against the enzyme amount applied is undesirably decreased.

The process for the production of maltooligosaccharides using the above immobilized enzyme will hereinafter be explained.

As described above, the present inventors have found that when starch hydrolysates are further hydrolyzed by feeding it to a reactor charged with the above immobilized enzyme, under specific conditions which are determined depending on the activity of the immobilized enzyme, maltooligosaccharides can be produced efficiently. That is, the process for the production of maltooligosaccharides of the present invention comprises feeding liquefied starch to a reactor charged with the above immobilized maltooligosaccharide-forming amylase under such conditions that the weight based space velocity per unit activity of the immobilized enzyme is $1 \times 10^{-4}$ to $3 \times 10^{-2}$ $hr^{-1}$ $(IU/g)^{-1}$.

As the starch to be used in the process of the present invention, various kinds of starch can be used. Usually potato starch, sweet potato starch, corn starch, waxy corn starch and cassava starch are used. The dextrose equivalent (DE) of the liquefied starch to be supplied to the reactor is usually from 1 to 35 and preferably from 5 to 20. If the DE of the liquefied starch is not more than 1 in the case of waxy corn starch, or not more than 10 in the case of other kinds of starch, retrogradation occurs seriously. To prevent this retrogradation, it is necessary to heat to and maintain at temperatures higher than 70—80°C. This is disadvantageous from a viewpoint of process. On the other hand, if DE is more than 35, the amounts of low molecular weight saccharides such as glucose and maltose are increased and the yield of the desired maltooligosaccharide is undesirably decreased.

The above starch can be liquefied by any suitable method. Usually the liquefaction is carried out by treating starch with bacterial liquefying α-amylase or acids such as hydrochloric acid.

Maltooligosaccharides include maltotriose, maltotetraose, maltopentaose, and maltohexaose. The term "maltooligosaccharide-forming amylases" as used herein means amylases capable of specifically forming the above various maltooligosaccharides.

The properties of the immobilized maltooligosaccharide-forming amylase can be improved by treating with suitable multifunctional cross-linking agent, such as glutaraldehyde. Also these enzymes can be used in the process of the present invention.

As a result of extensive investigations on conditions for efficient production of maltooligosaccharides using various immobilized enzymes, it has been found that the following factors, for example, exert great influences on the efficient production of maltooligosaccharides, the kind and concentration of a substrate used, and the amount of the substrate fed, physical properties of the carriers, the amount of the immobilized enzyme, the amount of the immobilized enzyme charged to a reactor, the temperature of the reaction system, pH and so forth.

As a result of investigations, it has been found that maltooligosaccharides can be produced efficiently when these factors are set to the conditions as defined below. That is, the process of the present invention relates to a process for production of maltooligosaccharides with high efficiency which comprises charging an immobilized maltooligosaccharide-forming amylase in a reactor and feeding the above starch hydrolyzate to the reactor under conditions that the weight based space velocity per unit activity of the immobilized enzyme is $1 \times 10^{-4}$ to $3 \times 10^{-2}$ $hr^{-1}$ $(IU/g)^{-1}$ and preferably $3 \times 10^{-4}$ to $1 \times 10^{-2}$ $hr^{-1}$ $(IU/g)^{-1}$.

The weight based space velocity per unit activity of the immobilized enzyme is determined as follows.

The same immobilized maltooligosaccharide-forming amylase (10 mg (wet)) as to be charged to a reactor is added to 0.5 ml of a 10 mM phosphate buffer (pH 7.0) in a 50 ml Erlenmeyer flask and mixed therewith thoroughly. Then, 5.0 ml of the same substrate as to be fed to the reactor (other conditions such as the kind and concentration of starch are also the same) is added, and the flask is shaken at the same temperature as to be employed in the reactor by the use of a reciprocal shaker under the condition of 120 strokes/min and 4 centimeters (cm) width to cause an enzyme reaction. To determine the immobilized enzyme activity the reducing sugar thus formed is measured by the Somogyi-Nelson method or

5

maltooligosaccharide formed is directly measured with an analytical apparatus such as high performance liquid chromatography. This exhibited activity is referred to as "A" and expressed in IU/g-carrier.

With the amount of the immobilized maltooligosaccharide-forming amylase charged to the reactor as "B" (g (wet)) and the amount (dry solid base) of the liquefied starch fed to the reactor as "C" (g-dry solid/ hr), the weight based space velocity per unit activity of the immobilized enzyme is calculated from the following equation.

$$\frac{C}{A \times B}(hr^{-1} (IU/g)^{-1})$$

If DE of the liquefied starch is larger than required the liquefied starch, the starting material, would contain the desired maltooligosaccharide and saccharides having molecular weight smaller than that of the desired maltooligosaccharide and, therefore, it is more practical that the C value excluding such smaller saccharides is employed.

If the weight based space velocity per unit activity is greater than $3 \times 10^{-2}$ $hr^{-1}$ $(IU/g)^{-1}$; that is, the reaction time in the reactor is short, the hydrolysis reaction is carried out only insufficiently and thus the yield of the maltooligosaccharide is undesirably low. On the other hand, if the weight based space velocity per unit activity is smaller than $1 \times 10^{-4}$ $hr^{-1}$ $(IU/g)^{-1}$; that is, the reaction time in the reactor is long, as described in the reference as described hereinafter, the formed maltooligosaccharide is further decomposed, resulting in the formation of low molecular weight saccharides such as glucose and maltose. This formation of low molecular weight saccharides is not desired because not only the purity of the product is seriously decreased but also the efficiency in the purification/separation process is reduced.

In connection with the above excessive decomposition of maltooligosaccharide, it has been revealed as described by T. Nakakuki et al. in Carbohydr. Res., *128*, 297 (1984) that the maltooligosaccharide-forming amylase specifically produces the corresponding oligosaccharide (e.g., maltotriose, maltotetraose, maltopentaose and malthohexaose) at an earlier stage of the reaction but at a later stage, decomposes the product.

When immobilized maltooligosaccharide-forming amylase of the present invention is used, reaction conditions can be expected to extend as compared with the native or mobile maltooligosaccharide-forming amylase. For example, when the immobilized maltooligosaccharide-forming amylase of the present invention is used, the temperature stability is extended to a higher side by about 10°C, and pH stability is extended to an acidic side by about 1. Further, the optimum temperature shift to a higher side by 10—15°C, and the optimum pH range is extended to an acidic side. That is, the properties of the enzyme are greatly improved by the immobilization of the present invention, and the desired reaction can be carried out under greatly advantageous conditions over those using the native or mobile enzyme.

In production of the maltooligosaccharide by the process of the present invention, various methods can be employed depending on the desired purity of the maltooligosaccharide product. For example, a maltooligosaccharide having a purity of about 20 to 60% can be produced by feeding liquefied starch to a reactor with the aforementioned immobilized maltooligosaccharide-forming amylase, under the aforementioned conditions. A maltooligosaccharide having a higher purity (60—100%) can be obtained by further purifying the product from the above reactor. This purification/separation can be carried out by various known techniques. For example, membrane ultrafiltration, gel filtration chromatography, cation exchange resin column chromatography and carbon column chromatography are effective. It is also possible that part or the whole of the starch remaining undecomposed as obtained after the purification/separation process is returned to the reactor charged with the immobilized maltooligosaccharide-forming amylase to increase the yield of the desired maltooligosaccharide per the liquefied starch. Furthermore, part or the whole of the undecomposed compound can be used maltooligosaccharide-forming amylase as limit dextrin without any modification.

The present invention is described below in greater detail with reference to the following examples.

Example 1

In this example, maltotetraose-forming amylase (from *Pseudomonas stutzeri*; specific activity: 80.8 IU/ mg · protein) was used. This enzyme was immobilized using carriers shown in Table 1.

A carrier (0.2 g) and 2.0 ml of a 50 mM phosphate buffer (pH 7.0) containing 100 IU of the enzyme were placed in a 50-ml Erlenmeyer flask, which was then shaken at a room temperature for 1 hour under the conditions of 120 strokes/min and 4 cm width to immobilize the enzyme on the carrier. The mixture was filtrated, and the filtrate was removed. The carrier was thoroughly washed with a 10 mM phosphate buffer (pH 7.0) until no protein was eluted, to thereby obtain an immobilized enzyme.

The affinity and activity of this immobilized enzyme was measured by the methods as described above. The results are shown in Table 1 along with the specific surface area of the carrier.

TABLE 1

| Carrier | Affinity (%) | Specific surface area of pores having diameter of $10^{-8}$ m or more (m²/g) | Exhibited activity (IU/g) |
|---|---|---|---|
| Duolite adsorption resin | | | |
| "ES-8610" | 98.3 | 69 | 275 |
| "S-761" | 96.7 | 80 | 252 |
| "S-762" | 99.1 | 59 | 406 |
| "ES-771" | 99.1 | 159 | 347 |
| | | | |
| Duolite weakly basic anion exchange resin | | | |
| "A-562" | 98.2 | 61 | 171 |
| "A-7" | 98.3 | 74 | 187 |
| | | | |
| Diaion adsorption resin | | | |
| "HP-10" | 98.3 | 83 | 219 |
| "HP-50" | 98.7 | 42 | 331 |
| | | | |
| Amberlite adsorption resin | | | |
| "XAD-7" | 96.8 | 57 | 181 |
| "XAD-8" | 98.9 | 74 | 310 |

Comparative Example 1

Immobilized enzymes were obtained in the same manner as in Example 1 except that carriers having poor affinity as shown in Table 2 were used.

These immobilized enzymes were evaluated in the same manner as in Example 1. The results are shown in Table 2.

Comparative Example 2

Immobilized enzymes were obtained in the same manner as in Example 1 except that carriers having insufficient physical properties of specific surface area were used.

These immobilized enzymes were evaluated in the same manner as in Example 1. The results are shown in Table 3.

TABLE 2

| Carrier | Affinity (%) | Specific surface area of pores having diameter of $10^{-8}$ m or more (m²/g) | Specific surface area of pores having diameter of $4 \times 10^{-8}$ m or more (m²/g) | Exhibited activity (IU/g) |
|---|---|---|---|---|
| Duolite adsorption resin | | | | |
| "ES-865" | 2.2 | 91 | 28 | 2 |
| | | | | |
| Amberlite adsorption resin | | | | |
| "XAD-2" | 57.9 | 60 | 11 | 26 |
| "XAD-4" | 24.4 | 93 | 22 | 11 |

TABLE 3

| Carrier | Affinity (%) | Specific surface area of pores having diameter $10^{-8}$ or more (m²/g) | Specific surface area of pores having diameter of $4 \times 10^{-8}$ m or more (m²/g) | Exhibited activity (IU/g) |
|---|---|---|---|---|
| Duolite weakly basic anion exchange resin | | | | |
| "A-374" | 99.3 | 6 | 6 | 3 |

Example 2

In this example, maltopentaose-forming amylase (from *Bacillus licheniformis*; specific activity: 40.9 IU/mg · protein) was used as an enzyme. This enzyme was immobilized in the same manner as in Example 1 using carriers as shown in Table 4.

The affinities and activities of these immobilized enzymes were measured in the same manner as in Example 1. The exhibited activity was measured at 40°C. The results are shown in Table 4.

TABLE 4

| Carrier | Affinity (%) | Specific surface area of pores having diameter of $10^{-8}$ m or more (m²/g) | Exhibited activity (IU/g) |
|---|---|---|---|
| Duolite adsorption resin | | | |
| "ES-8610" | 100 | 69 | 64 |
| "S-761" | 100 | 80 | 96 |
| "S-762" | 100 | 59 | 119 |
| "ES-771" | 99.3 | 159 | 104 |
| Duolite weakly basic anion exchange resin | | | |
| "S-587" | 96.9 | 106 | 126 |
| "A-561" | 92.0 | 93 | 117 |
| "A-562" | 100 | 61 | 95 |
| "A-7" | 86.9 | 74 | 134 |
| Diaion adsorption resin | | | |
| "HP-10" | 100 | 146 | 52 |
| "HP-50" | 99.6 | 42 | 78 |
| Amberlite adsorption resin | | | |
| "XAD-7" | 84.0 | 57 | 90 |

Comparative Example 3

Immobilized enzymes were obtained in the same manner as in Example 2 except that carriers having poor affinity as shown in Table 5 were used. The results are shown in Table 5.

TABLE 5

| Carrier | Affinity (%) | Specific surface area of pores having diameter of $10^{-8}$ m or more (m²/g) | Exhibited activity (IU/g) |
|---|---|---|---|
| Duolite adsorption resin | | | |
| "S-863" | 20.9 | 80 | 4.0 |
| "ES-866" | 12.7 | 72 | 3.0 |
| Duolite weakly basic anion exchange resin | | | |
| "A-368" | 12.0 | 50 | 2.9 |
| Diaion adsorption resin | | | |
| "SDA-03" | 22.6 | 66 | 9.9 |
| Amberlite adsorption resin | | | |
| "XAD-4" | 28.5 | 93 | 7.7 |

Comparative Example 4

Immobilized enzymes were obtained in the same manner as in Example 2 except that carriers having insufficient physical properties of specific surface area as shown in Table 6 were used. The results are shown in Table 6.

8

TABLE 6

| Carrier | Affinity (%) | Specific surface area of pores having diameter of $10^{-8}$ m or more ($m^2/g$) | Exhibited activity (IU/g) |
|---|---|---|---|
| Diaion weakly basic anion exchange resin | | | |
| "PA-406" | 100 | 12 | 9.1 |
| "PA-408" | 99.5 | 13 | 5.1 |
| "PA-416" | 99.9 | 13 | 3.3 |
| "PA-418" | 98.9 | 15 | 2.7 |

Example 3

Maltotetraose-forming amylase (from *Pseudomonas stutzeri*; specific activity: 80.8 IU/mg · protein) was immobilized using an adsorption resin Diaion "SP-206" as a carrier.

The carrier (20 g) and 150 ml of a 50 mM phosphate buffer (pH 7.0) containing 20,000 IU of the enzyme were placed in a 500-ml Erlenmeyer flask, which was then shaken at a room temperature for 1 hour under the conditions of 120 strokes/min and 4 cm width to immobilize the enzyme on the carrier. The mixture was filtered, and the filtrate was removed. The carrier was fully washed with a 10 mM phosphate buffer (pH 7.0) until no protein was eluted, to thereby obtain an immobilized enzyme having exhibited activity of 137 IU/g.

The above immobilized enzyme (20 g) was packed in a column having a diameter of 27 mm and a length of 130 mm. Liquefied 20 w/w % waxy corn starch (DE 7.5) was fed to the column reactor at a rate of 40 g/hr, that is, under condition of weight based space velocity per unit activity $2.92 \times 10^{-3}$ and at a temperature of 45°C. The composition of the reaction product after 360 hours is shown in Table 7. The half-life of the enzyme in the reaction was 1,680 hours.

Example 4

Immobilized maltotetraose-forming amylase (exhibited activity: 1,39 IU/g) was prepared using an adsorption resin Duolite "ES-8610" as a carrier in the same manner as in Example 3.

The immobilized maltotetraose-forming amylase (10 g) was packed in a reactor, and 16.7 w/w % soluble starch (produced by Wako Junyaku Co., Ltd.) was fed to the reactor at a rate of 10.7 g/hr (as solids, 1.79 g/hr) and a temperature of 40°C (weight based space velocity per unit activity: $1.3 \times 10^{-3}$ $hr^{-1}$ $(IU/g)^{-1}$. The operation results are shown in the Figure.

As apparent from the Figure, the stable operation could be continued for more than 1,500 hours.

The reaction product from the outlet of the column reactor was divided into two portions, Product 1 containing maltotetraose as the major component and Product 2 consisting of the residual matter, by the use of a membrane ultrafiltration apparatus (molecular weight cut off: 1,000). The saccharide composition of Product 1 after 1,500 hours is shown in Table 7.

Comparative Example 5

Immobilized maltotetraose-forming amylase (exhibited activity: 167 IU/g) was prepared using an adsorption resin Diaion "HP'50" in the same manner as in Example 3.

The immobilized maltotetraose-forming amylase (7 g) was packed in a reactor, and liquefied 4.76 w/w % waxy corn starch (DE 8) was fed to the reactor at a temperature of 40°C and a rate of 1.21 g/hr (weight based space velocity per unit activity: $4.9 \times 10^{-5}$ hr $(IU/g)^{-1}$). The saccharide composition of the reaction product is shown in Table 7.

Comparative Example 6

Immobilized maltotetraose-forming amylase (exhibited activity: 49 IU/g) was prepared using an adsorption resin Diaion "HP-50" in the same manner as in Example 3.

The immobilized maltotetraose-forming amylase (7 g) was packed in a reactor, and liquefied 23.1 w/w % waxy corn starch (ED 8) was fed to the reactor at a temperature of 40°C and a rate of 56.0 g/hr (weight based space velocity: $3.8 \times 10^{-2}$ hr $(IU/g)^{-1}$). The saccharide composition of the reaction product is shown in Table 7.

TABLE 7
Composition of reaction product (%)

| | Example | | Comparative Example | |
| --- | --- | --- | --- | --- |
| | 3 | 4 | 5 | 6 |
| $G_1$—$G_3$ [*1] | 15.7 | 8.9 | 81.3 | 5.4 |
| $G_4$ [*2] | 43.2 | 90.0 | 12.7 | 16.2 |
| $G_5^+$ [*3] | 41.1 | 1.1 | 6.0 | 78.4 |

Note
[*1] Glucose, maltose and maltotriose.
[*2] Maltotetraose.
[*3] Maltopentaose and higher molecular weight saccharides.

In accordance with the present invention, maltooligosaccharide-forming amylases can be immobilized efficiently and the activity of the immobilized enzyme is sufficiently high. Furthermore the activity of the immobilized enzyme can be maintained for a long time.

Thus the immobilized enzyme of the present invention permits commercial production of maltooligosaccharides.

In production of maltooligosaccharides according to the process of the present invention, the activity of the immobilized enzyme can be maintained for a long time and the desired maltooligosaccharide can be produced efficiently by feeding liquefied starch under the conditions as defined in the present invention. Furthermore, high purity maltooligosaccharide can be obtained. In practice of the process of the present invention, the reaction temperature and pH can be chosen from wide ranges.

Thus the process of the present invention is useful for commercial production of maltooligosaccharide.

## Claims

1. Immobilized maltooligosaccharide-forming amylase selected from the group consisting of maltotriose-forming amylase, maltotetraose-forming amylase, maltopentaose-forming amylase and maltohexaose-forming amylase, which is immobilized on a porous carrier wherein the carrier is porous synthetic resin selected from the group consisting of phenol-based adsorption resin, styrene/divinylbenzene-based adsorption resin, triazine-based adsorption resin, and acrylate-based adsorption resin, having following characteristics:
(a) an affinity of enzyme to carrier is 80 percent or more; and
(b) a specific surface area of pores having a diameter of $10^{-8}$ m or more is from 20 to 300 square meters per gram.

2. The immobilized maltooligosaccharide-forming amylase of claim 1, wherein the carrier is a carrier having a particle diameter of from 0.05 to 1.5 millimeters.

3. A process for producing maltooligosaccharide which comprises feeding liquefied starch to a reactor charged with an immobilized maltooligosaccharide-forming amylase as defined in claim 1 or 2, at a weight based space velocity per unit activity of the immobilized enzyme of $1 \times 10^{-4}$ to $3 \times 10^{-2}$ hr$^{-1}$ (IU/g)$^{-1}$.

## Patentansprüche

1. Immobilisierte Maltooligosaccharide bildende Amylase, ausgewählt aus der Gruppe, die aus Maltotriose bildender Amylase, Maltotetraose bildender Amylase, Maltopentaose bildender Amylase und Maltohexaose bildender Amylase besteht, die auf einem porösen Träger immobilisiert ist, worin der Träger ein poröses synthetisches Harz ist, ausgewählt aus der Gruppe, die aus auf Phenol basierendem Adsorptionsharz, Styrol/Divinylbenzol basierendem Adsorptionsharz, Triazin basierendem Adsorptionsharz und Acrylat basierendem Adsorptionsharz besteht, mit folgenden Eigenschaften:
(a) eine Affinität des Enzyms zum Träger ist 80% oder mehr; und
(b) eine spezifische Mantelfläche von Poren mit einem Durchmesser von $10^{-8}$ m oder mehr beträgt von 20 bis 300 m$^2$ pro Gramm.

2. Immobilisierte Maltooligosaccharide bildende Amylase von Anspruch 1, in welcher der Träger ein Träger ist, der einen Teilchendurchmesser von 0,05 bis 1,5 mm hat.

3. Verfahren zur Herstellung von Maltooligosaccharid, das ein Zuführen verflüssigter Stärke in einen Reaktor, beladen mit einer immobilisierten Maltooligosaccharide bildender Amylase wie in Anspruch 1 oder 2 definiert, bei einer auf Gewicht basierenden Raumgeschwindigkeit pro Einheit Aktivität des immobilisierten Enzyms von $1 \times 10^{-4}$ bis $3 \times 10^{-2}$ hr$^{-1}$ (IU/g)$^{-1}$, beinhaltet.

**Revendications**

1. Amylase immobilisée formant un maltooligosaccharide choisie dans le groupe constitué par une amylase formant du maltotriose, une amylase formant du maltotétrose, une amylase formant du maltopentose et une amylase formant du maltohex, qui est immobilisée sur un support poreux, le support étant une résine synthétique poreuse choisie dans le groupe constitué par les résines d'adsorption à base de phénol, les résines d'adsorption à base de styrène/divinylbenzène, les résines d'adsorption à base de triazine et les résines d'adsorption à base d'acrylate, ayant les caractéristiques suivantes:

(a) une affinité de l'enzyme pour le support de 80% ou plus; et

(b) une surface spécifique des pores ayant un diamètre de $10^{-8}$ m ou plus de 20 à 300 mètres carrés par gramme.

2. Amylase immobilisée formant un maltooligosaccharide selon la revendication 1, dont le support a un diamètre des particules de 0,05 à 1,5 millimètre.

3. Procédé pour produire un maltooligosaccharide qui consiste à alimenter, avec un amidon liquéfié, un réacteur chargé d'une amylase immobilisée formant un maltooligosaccharide comme défini dans la revendication 1 ou 2, avec une vitesse spatiale pondérale par unité d'activité de l'enzyme immobilisée de $1 \times 10^{-4}$ à $3 \times 10^{-2}$ h$^{-1}$ (UI/g)$^{-1}$.

FIG.1